# NOUVEAU FASCICULE DE BREVET EUROPEEN

(11) **EP 0 432 490 B2**
(45) Date de publication et mention de la décision concernant l'opposition: **16.05.2001**
(45) Mention de la délivrance du brevet: 30.08.1995
(21) Numéro de dépôt: 90121752.1
(22) Date de dépôt: 14.11.1990
(51) Int. Cl.: A61K 39/07, A23C 9/12

(54) **Agent immunostimulant**
Immunostimulierendes Mittel
Immunostimulant agent

(30) Priorité: 13.12.1989 CH 448489
(43) Date de publication de la demande: 19.06.1991
(73) Titulaire: SOCIETE DES PRODUITS NESTLE S.A., 1800 Vevey (CH)
(72) Inventeur: Link, Harriet, CH-1800 Vevey (CH); Pahud, Jean-Jacques, CH-1801 Mont-Pelerin (CH)

(56) Documents cités:
- EP-A- 0 089 266
- FR-A- 2 520 379
- GB-A- 2 046 759
- SU-A- 1 465 003
- BIOTHERAPY, vol. 1, no. 3, 8 décembre 1989, pages 169-177; K. NOMOTO et al.:"Augmentation of resistance of mice to bacterial infection by a polysaccaride-peptidoglycan complex (PSPG) extracted from Lactobacillus casei"
- Namba et al., Infect. and Immunity 31(2), 580-83 (1981)
- Bezrukov et al., Chem of Pept and Proteins 3, 85-98 (1986)

## Description

La présente invention a pour objet un agent immunostimulant et un procédé de préparation d'un tel agent, ainsi qu'une utilisation de cet agent et/ou de ce procédé dans le domaine des laits fermentés.

FR-B-1.514.949 (1968) décrit un procédé de préparation d'un agent anticarcinogène, dans lequel on cultive un lactobacille, notamment le Lactobacillus bulgaricus, on l'hydrolyse avec une enzyme protéolytique et l'on extrait de l'hydrolysat un produit riche en protéine et en acide ribo-nucléique qui présente une activité anticarcinogène.

On connaît par ailleurs un procédé de préparation d'un produit diététique à base de lait pour les enfants, dans lequel on supplémente un lait, acidifié par Lactobacillus acidophilus, avec des oligoéléments, des vitamines et une solution de lysozyme. Ce dernier est sensé remplacer le lysozyme présent dans le lait maternel et améliorer la conservation, le goût et la texture de ce produit diététique.

FR 2520379 décrit un procédé de préparation d'un disaccharide-tripeptide et d'un disaccharide-tétrapeptide possédant des propriétés immunostimulantes, par hydrolyse des parois cellulaires de bactéries telles que *Lactobacillus plantarum* et *Corynebacterium diphteriae*, avec une endo-N-acétylmuramidase de *Streptomyces globisporus* ou de *Streptomyces nitrosporeus.* L'agent immnunostimulant ainsi obtenu est un produit purifié à usage pharmaceutique à administrer par voie parentérale.

Biotherapy, 1(3), 169-177 (1989) décrit la préparation, par digestion de *Lactobacillus casei* avec une N-acétylmuramidase, d'un complexe polysaccharidepeptidoglycane (PSPG) capable d'augmenter la résistance de souris aux infections. Ce PSPG présente un poids moléculaire supérieur à 30 000 et est administré aux souris par injection intrapéritonéale.

Bezrukov et al., Chemistry of Peptides and Proteins 3, 85-98 (1986) décrivent la "blastolysine", un hydrolysat résultant de l'action du lysozyme sur la paroi cellulaire de *Lactobacillus bulgaricus* et analysent cet hydrolysat. Un fractionnement par passages successifs sur deux colonnes de gel permet d'isoler une fraction présentant une activité anticarcinogène et immunostimulante dont le principe actif est un N-acétyl-muramyl-peptide.

Ivanov et al., Pure & Appl. Chem 59 No 3, 317-324 (1987) reprennent pour l'essentiel le contenu de l'article Bezrukov et al. ci-dessus et y ajoutent la description de la synthèse de N-acétyl-muramyl-peptides présentant une activité immunostimulante.

SU-A1-1465003 (1989) décrit un procédé de préparation d'un lait adapté aux besoins des nourrissons, dans lequel on fermente un milieu lacté avec un microorganisme lactique, notamment un *Lactobacillus bulgaricus*, et, en fin d'acidification, on ajoute du lysozyme et l'on laisse refroidir de la température de fermentation à la température ambiante, puis on refroidit brusquement à température de réfrigération.

Pahud et al., Biochem. J. 201, 661-664 (1982) concerne une investigation purement biochimique sur la présence éventuelle de lysozyme dans les sécrétions bovines.

Namba et al., Infection and Immunity 31 No. 2, 580-583 (1981) est consacré à l'étude de l'effet immunostimulant chez le cobaye de l'administration orale de lysozyme ou de parois cellulaires de bactéries intestinales digérées par le lysozyme, en l'occurrence un lysozyme de blanc d'oeuf.

La présente invention a pour but de proposer une utilisation dans le domaine des laits fermentés d'un agent immunostimulant efficace, obtenable à partir de la bactérie lactique *Lactobacillus bulgaricus.*

A cet effet, la présente invention comprend une utilisation dans un lait fermenté, notamment dans un yogourt, ou dans un produit lactosérique, d'un agent immunostimulant comprenant des N-acétyl-muramyl-peptides dérivés, par hydrolyse avec du lysozyme, de peptidoglycanes de la paroi de cellules de *Lactobacillus bulgaricus.*

De même, la présente invention comprend une utilisation dans la préparation d'un lait fermenté, notamment d'un yogourt, ou dans la préparation d'un produit lactosérique, d'un procédé de préparation d'un agent immunostimulant, dans lequel on hydrolyse avec du lysozyme les peptidoglycanes de la paroi de cellules de *Lactobacillus bulgaricus*, pour en dériver des N-acétyl-muramyl-peptides. On a constaté en effet qu'un tel agent présente un effet stimulant remarquable de la réponse immunitaire, notamment de la réponse immunitaire aux bactéries entéropathogènes gram négatives, telles qu'E.coli par exemple.

Un tel agent se prête particulièrement bien à une utilisation dans un lait fermenté, notamment dans un yogourt ou dans un produit lactosérique.

De même, un tel procédé se prête bien à une utilisation, autrement dit peut être directement réalisé dans le cadre de la préparation d'un lait fermenté, notamment d'un yogourt, ou dans la préparation d'un produit lactosérique.

La présente invention s'étend donc à ces utilisations.

Dans le présent exposé, l'effet immunostimulant du présent produit est estimé de manière qualitative sur le modèle de la souris. On sait que, si les résultats de tels essais ne sont pas directement transposables à l'homme, ils n'en fournissent pas moins des indications utiles.

Dans le présent exposé également, l'expression "bactéries lactiques sensibles au lysozyme" est utilisée dans le sens de bactéries lactiques dont la paroi cellulaire comprend des peptidoglycanes hydrolysables par le lysozyme, autrement dit dégradables en N-acétyl-muramyl-peptides sous l'action du lysozyme.

Pour mettre en oeuvre la présente utilisation du procédé, on peut notamment utiliser une culture pure de Lactobacillus bulgaricus ou une culture mixte de Lactobacillus bulgaricus et de Streptococcus thermophilus, autrement dit une culture de yogourt, telle qu'on en trouve dans le commerce, par exemple.

On peut utiliser en particulier une culture ou une suspension contenant 10⁶-10¹⁰ cellules de L.bulgaricus par ml. Pour produire une quantité désirée de cellules en concentration suffisante, on peut inoculer un milieu de culture adéquat avec une culture d'ensemencement de ladite bactérie et incuber à une température et à un pH favorables à la croissance de la bactérie, durant un temps suffisant pour rétablir dans le milieu de culture une concentration de cellules comparable à celle de la culture d'amorçage. Ledit milieu de culture adéquat peut être un milieu lacté, tel qu'un lait de vache écrémé ou non, un lactosérum, un perméat d'ultrafiltration, ou un milieu synthétique, par exemple.

Pour préparer une culture de L.bulgaricus, on peut inoculer un lait de vache, ou un milieu synthétique tel que le milieu MRS (Difco), par exemple, avec une culture d'ensemencement de ce microorganisme et incuber durant 3-6 h à 37-45°C. Si l'on incube durant moins de 3 h ou à moins de 37°C, on risque de demeurer dans la phase dite latente, autrement dit la phase de démarrage de la multiplication du microorganisme, ou de ne pas être assez longtemps engagé dans la phase dite exponentielle, autrement dit la phase de multiplication exponentielle du microorganisme.

Par contre, si l'on incube durant plus de 6 h, on risque d'entrer dans la phase stationnaire, autrement dit la phase où la multiplication du microorganisme cesse et au cours de laquelle la composition de la paroi cellulaire peut se modifier de manière défavorable. Enfin, au-delà de 45°C la température n'est plus favorable à la croissance de L.bulgaricus.

On réalise donc ladite hydrolyse avec du lysozyme. Celui-ci peut provenir de toute source adéquate, notamment de la présure, en particulier de la présure de ruminant (surtout bovin), ou du blanc d'oeuf, sous forme pure ou non. Dans une forme d'exécution particulière des présentes utilisations, on utilise en effet la présure elle-même, sans en avoir séparé le lysozyme.

On peut réaliser l'hydrolyse desdits peptidoglycanes de la paroi de cellules de L.bulgaricus à 15 -37°C, durant 1-48 h, de préférence 12-24 h, à l'aide de 1-500 µg de lysozyme par ml d'une culture contenant 10⁶ -10¹⁰ cellules de ces bactéries par ml, par exemple, à un pH favorable à l'action du lysozyme utilisé. Ce pH est d'environ 4-6 pour un lysozyme de présure et d'environ 6-8 pour un lysozyme de blanc d'oeuf, par exemple.

Pour réaliser les utilisations selon la présente invention, on peut d'une part ajouter le présent agent, tel quel ou sous forme d'extrait aqueux, à un lait fermenté tel qu'un yogourt liquide ou brassé, ou à un produit lactosérique tel qu'une boisson à base de lactosérum. La quantité d'agent ajouté peut correspondre à la quantité de N-acétyl-muramyl-peptides dérivés des parois de 10⁶ -10¹⁰ cellules de L.bulgaricus par ml dudit lait fermenté ou dudit produit lactosérique, par exemple.

On peut d'autre part réaliser ladite hydrolyse dans un lait fermenté, notamment par L.bulgaricus seul ou en combinaison avec S.thermophilus, ou dans un lactosérum, par exemple. Dans le cas du lait fermenté, on peut ajouter le lysozyme dans le lait avant, pendant ou après sa fermentation, à raison de environ 1-500 *µ*g de lysozyme par ml de lait, par exemple. Dans le cas du lactosérum, on peut lui ajouter lesdites cellules de L.bulgaricus, on peut même, le cas échéant, les y cultiver, et on peut ajouter, si nécessaire, du lysozyme.

Les exemples ci-après sont présentés à titre d'illustration de la présente invention. Les pourcents y sont donnés en poids, sauf indication contraire.

L'effet immunostimulant, autrement dit l'effet stimulant de la réponse immunitaire, est estimé par l'intermédiaire d'une détermination de concentrations en anticorps spécifiques, sur la souris, par le test ELISA.

Ce dernier test, bien connu de l'homme du métier, consiste à doser une enzyme marquant un anti-anticorps qui se lie à un anticorps spécifique d'une immunoglobuline, telle que IgG, IgM ou IgA, qui se lie à un antigène, tel qu'un antigène bactérien, par exemple, lui-même fixé sur un support insoluble en milieu aqueux.

### Exemple comparatif (i)

On prépare un milieu de culture synthétique, consistant en un milieu MRS (Difco Lab., USA) modifié, présentant la composition suivante (exprimée en g de matière sèche par litre de milieu):

| | |
|---|---|
| hydrolysat de protéines | 10 |
| extrait de viande | 10 |
| extrait de levure | 5 |
| glucose | 20 |
| lactose | 10 |
| émulsifiant | 1 |
| citrate d'ammonium | 2 |
| acétate de sodium | 5 |
| MgSO₄ | 0,1 |
| MnSO₄ | 0,05 |
| K₂HPO₄ | 2 |

Ce milieu de culture synthétique présente un pH de 6,5. On le stérilise durant 15 min à 121°C. On l'inocule avec une culture d'ensemencement de Lactobacillus bulgaricus, en l'occurence une culture du L.bulgaricus NCDO 1489 (National Culture of Food Bacteria, AFRC Institute of Food Research, Reading Laboratory, Shinfield, Reading, RG2 9AT UK).

On incube à 40°C durant 6 h. On sépare la biomasse, on la lave et on la sépare en deux parties A et B. On met la partie A en suspension dans un tampon phosphate 0,02 M à pH 7 et la partie B en suspension dans un tampon acétate 0,01 M à pH 5, à raison de 2.10⁹ cellules du microorganisme par ml.

On hydrolyse les cellules de L.bulgaricus de la suspension A avec un lysozyme de blanc d'oeuf du commerce (Calbiochem, Allemagne fédérale). On hydrolyse les cellules de L.bulgaricus de la suspension B avec un lysozyme de présure extrait et purifié comme décrit par J.J.Pahud et al. dans Biochem.J.201, 661-664 (1982).

Pour ce faire, on ajoute 16 µg de lysozyme par ml de suspension et l'on incube à 25°C, durant 18 h. On centrifuge et on filtre le surnageant sur une membrane présentant des pores de 0,45 µm de diamètre.

On obtient ainsi deux agents immunostimulants A et B, sous forme de solutions stériles ou extraits contenant les N-acétyl-muramyl-peptides dérivés, par hydrolyse avec du lysozyme de blanc d'oeuf (A) ou de présure (B), des parois cellulaires de 2.10⁹ cellules de L.bulgaricus par ml.

On vérifie l'effet immunostimulant de ces agents A et B sur la souris de race "Swiss white" (Tuttlingen, Allemagne fédérale), selon le protocole expérimental suivant:

Six groupes numérotés de 1 à 6 comprenant chacun 6 souris de 18-20 g reçoivent une diète normale et de l'eau à volonté. A compter du début de l'expérience au jour dit O, on procède à des intubations orales aux jours 1, 3, 5, 15, 17, 19 et 30. La diète et l'eau sont retirées aux souris 3 h avant chacune de ces intubations et rétablis immédiatement après. Des sera sont prélevés des souris aux jours 0, 29 et 40. Un lavage intestinal est pratiqué au jour 40.

Chaque intubation orale est réalisée avec une dose de 0,5 ml par souris. La composition de ces doses est la même pour les souris du même groupe, mais elle varie de groupe en groupe conformément au tableau 1 ci-après. Dans ce tableau, la rubrique "vaccin" indique que l'on a mis en suspension, par dose, 5.10⁹ cellules d'Eschericia coli 0111:K58 dont une moitié ont été inactivées durant 1 h à 100°C et l'autre moitié ont été inactivées avec une solution de glutaraldéhyde à 0,05% (Hilpert et al., Food and Immunology, 1977, Ed.L.Hambraeux, L.A.Hanson, H.Mc Farlane, Swedish Nutrition Foundation Symposium XIII, 182-196). Les lettres CT désignent la toxine du choléra, un agent immunostimulant bien connu de l'homme du métier.

L'expression "eau physiologique" désigne de l'eau distillée contenant 0,9% de NaCI.

**TABLEAU I**

| Groupe | Liquide de base | Substances ajoutées | | |
|---|---|---|---|---|
| | | NaHCo₃ % | autres | |
| 1 | eau physiologique | 3 | - | - |
| 2 | " | 3 | - | 1 *µ*g CT |
| 3 | " | 3 | vaccin | - |
| 4 | " | 3 | vaccin | 1 µg CT |
| 5 | agent A | 3 | vaccin | - |
| 6 | agent B | 3 | vaccin | - |

On détermine par le test ELISA la concentration en anticorps IgG et IgM des sera, ainsi que la concentration en anticorps IgG et IgA des fluides de lavage intestinaux prélevés des souris. Le tableau Il ci-dessous présente les valeurs relatives obtenues. Les chiffres indiqués sont une moyennne géométrique des valeurs déterminées pour chacune des six souris d'un même groupe. Les astérisques indiquent que pour les chiffres ainsi marqués, la probabilité que l'on fasse erreur en prétendant qu'il sont différents du chiffre correspondant (même colonne) du groupe 3 (témoin, souris qui n'ont reçu que le vaccin seul) est inférieure à 5%. En d'autres termes, pour les chiffres marqués d'une astérisque, la valeur "p", déterminée par analyse de variance unilatérale, est plus petite que 0,05.

**TABLEAU II**

| Groupe | ex sera: | | | | ex fluide intestinal: | |
|---|---|---|---|---|---|---|
| | IgG | | IgM | | IgG | IgA |
| | j29 | j40 | j29 | j40 | j40 | j40 |
| 1 | 69 | 57 | 36 | 39 | 8 | 8 |
| 2 | 96 | 94 | 25 | 80 | 10 | 11 |
| 3 | 189 | 489 | 31 | 83 | 11 | 18 |
| 4 | 572^{*} | 759 | 113 | 123^{*} | 13 | 24 |
| 5 | 525^{*} | 828 | 104 | 141^{*} | 18 | 53^{*} |
| 6 | 397^{*} | 1362^{*} | 39 | 45 | 24^{*} | 72^{*} |

On constate donc que les sera prélevés aux jours 29 et 40 présentent une augmentation d'un facteur de environ 2-3 de la concentration en anticorps IgG pour les souris ayant reçu le vaccin et les agents A ou B (invention, groupes 5 et 6) ou CT (comparaison, groupe 4), par rapport aux souris qui n'ont reçu que le vaccin seul (témoin, groupe 3). L'augmentation de la concentration en anticorps IgM ne se constate par contre que pour l'agent A (invention, groupe 5) et CT (comparaison, groupe 3).

La concentration en anticorps IgG et IgA dans les fluides intestinaux des souris ayant reçu le vaccin et les agents A ou B (invention, groupes 5 et 6) présente également une augmentation très nette par rapport à celle des fluides intestinaux des souris ayant reçu le vaccin seul (témoin, groupe 3).

### Exemple comparatif (ii)

On prépare une biomasse de L.bulgaricus de la manière décrite à l'exemple comparatif (i). On la met en suspension dans un tampon acétate 0,01 M à pH 5, à raison de 1,6.10⁹ cellules du microorganisme par ml.

On ajoute à cette suspension 1% de présure liquide contenant 4% de lysozyme (Laboratoire Présure Granday, Beaune, France). On incube sous légère agitation durant 18 h à 25°C. On centrifuge et on filtre le surnageant sur une membrane à pores de 0,45 *µ*m de diamètre.

On obtient ainsi un agent immunostimulant C sous forme de solution stérile ou extrait contenant les N-acétyl-muramyl-peptides dérivés, par hydrolyse avec du lysozyme de présure, sous forme de la présure elle-même, des parois cellulaires de 1,6.10⁹ cellules de L.bulgaricus par ml.

On vérifie l'effet immunostimulant de cet agent C sur la souris de race "Swiss white" de manière semblable à celle décrite à l'exemple comparatif (i), à l'exception du fait que l'agent immunostimulant n'est par administré aux souris par intubation, mais qu'il est ajouté à l'eau dont elles s'abreuvent librement, à raison de 1 partie d'agent pour 50 parties d'eau. Durant toute l'expérience, on met 10 ml de lipide par 24 h à disposition des souris dont le besoin quotidien est d'environ 4-7 ml.

Quatre groupes de six souris chacun, numérotés de 1-4, reçoivent une diète normale. Deux de ces groupes reçoivent de l'eau physiologique à volonté. Les deux autres reçoivent de l'eau physiologique à laquelle on a ajouté l'agent immunostimulant C. En outre, les souris de deux de ces groupes reçoivent également un vaccin d'E.coli par intubation orale, de manière semblable à celle décrite au protocole expérimental de l'exemple comparatif (i). Le tableau III ci-après résume ce programme d'alimentation et/ou de vaccination des souris des différents groupes.

**TABLEAU III**

| Groupe | Liquide | Intubation |
|---|---|---|
| 1 | eau physiologique | - |
| 2 | eau physiologique + agent C | - |
| 3 | eau physiologique + agent C | vaccin |
| 4 | eau physiologique + agent C | vaccin |

On détermine par le test ELISA la concentration en anticorps IgG et IgM des sera, ainsi que la concentration en anticorps IgA des fluides de lavage intestinaux prélevés des souris. Le tableau IV ci-dessous présente les valeurs relatives obtenues.

**TABLEAU IV**

| Groupe | ex sera | | | | ex fluide intestinal |
|---|---|---|---|---|---|
| | IgG | | IgM | | IgA |
| | j29 | j40 | j29 | j40 | j40 |
| 1 | 77 | 68 | 54 | 101 | 2 |
| 2 | 96 | 78 | 37 | 66 | 3 |
| 3 | 1427 | 1213 | 71 | 94 | 25 |
| 4 | 2365 | 3180* | 219* | 360** | 31 |

| | | | | | |
|---|---|---|---|---|---|
| * valeur "p" = 0,065 | | | | | |
| ** valeur "p" = 0,014 | | | | | |

On constate donc que les sera prélevés aux jours 29 et 40 présentent une augmentation d'un facteur de environ 2-3, voire davantage, de la concentration en anticorps IgG et IgM pour les souris ayant reçu le vaccin et ayant bu une eau additionnée de l'agent C (invention, groupe 4), par rapport aux souris qui n'ont reçu que le vaccin seul (témoin, groupe 3). Par contre, on ne voit guère de différence entre les concentrations en anticorps IgA pour les souris de ces groupes 3 et 4.

### Exemple comparatif (iii)

On inocule un lait de vache avec 3% en volume d'une culture d'ensemencement pure du commerce, contenant environ 10⁸ cellules de Lactobacillus bulgaricus par ml. On incube 5 h à 40°C. On obtient une culture pure de L.bulgaricus contenant environ 10⁸ cellules de ce microorganisme par ml et présentant un pH de 4,6.

On ajoute à la culture 20 µg/ml de lysozyme de présure. On incube durant 12 h à 25°C.

On obtient ainsi un agent immunostimulant contenant par ml les N-acétyl-muramyl-peptides dérivés, par hydrolyse avec du lysozyme de présure, des peptidoglycanes des parois de 10⁸ cellules de L.bulgaricus.

### Exemple comparatif (iv)

On procède de la manière décrite à l'exemple comparatif (iii), jusqu'à l'obtention de la culture pure de L.bulgaricus.

On ajoute à la culture 1% de présure liquide contenant elle-même environ 4% de lysozyme. On incube durant 8 h à 20°C.

On obtient ainsi un agent immunostimulant contenant par ml les N-acétyl-muramyl-peptides dérivés, par hydrolyse avec le lysozyme de la présure, des peptidoglycanes des parois de 10⁸ cellules de L.bulgaricus.

### Exemple 1

On inocule un lait de vache avec 5% en volume d'une culture d'ensemencement mixte contenant, par ml, environ 5.10⁷ cellules de Lactobacillus bulgaricus et environ 2.10⁸ cellules de Streptococcus thermophilus obtenues à partir de cultures du commerce. On incube à 43°C durant 3 h. On obtient un yogourt contenant environ 2.10⁷ cellules de L.bulgaricus et environ 10⁸ cellules de S.thermophilus par ml.

On ajoute à ce yogourt 50 µg de lysozyme de présure par ml. On incube 12 h à 25°C et l'on refroidit à 4°C.

On obtient ainsi un produit du type yogourt présentant les propriétés d'un agent immunostimulant contenant par ml les N-acétyl-muramyl-peptides dérivés, par hydrolyse avec du lysozyme de présure, des peptidoglycanes des parois de 2.10⁷ cellules de L.bulgaricus.

### Exemple 2

On procède de la manière décrite à l'exemple 1, jusqu'à l'obtention du yogourt. On ajoute à ce yogourt 1% de présure liquide contenant 4% de lysozyme. On laisse incuber 24 h à 25°C.

On obtient ainsi un produit du type yogourt présentant les propriétés d'un agent immunostimulant contenant par ml les N-acétyl-muramyl-peptides dérivés, par hydrolyse avec du lysozyme de présure, des peptidoglycanes des parois de 2.10⁷ cellules de L.bulgaricus.

### Exemple 3

On centrifuge le produit obtenu à l'exemple 2 et l'on recueille le lactosérum. Celui-ci représente lui-même un agent immunostimulant riche en N-acétyl-muramyl-peptides dérivés, par hydrolyse avec du lysozyme de présure, de peptidoglycanes de parois cellulaires de L.bulgaricus.

### Exemple 4

On inocule un lactosérum doux de fromagerie avec une culture d'ensemencement de Lactobacillus bulgaricus. On incube 6 h à 42°C.

On refroidit à 25°C. On ajoute 1% de présure contenant elle-même 4% de lysozyme. On incube 18 h à 25°C.

On obtient un agent immunostimulant riche en N-acétyl-muramyl-peptides dérivés, par hydrolyse avec du lysozyme de présure, de peptidoglycanes de parois cellulaires de L.bulgaricus.

## Revendications

1. Utilisation dans un lait fermenté, notamment dans un yogourt, ou dans un produit lactosérique, d'un agent immunostimulant comprenant des N-acétyl-muramyl-peptides dérivés, par hydrolyse avec du lysozyme, de peptidoglycanes de la paroi de cellules de *Lactobacillus bulgaricus*.

2. Utilisation selon la revendication 1, dans laquelle le lysozyme est un lysozyme de présure, sous forme purifiée ou sous forme de la présure elle-même.

3. Utilisation selon la revendication 1, dans laquelle, pour préparer des cellules de *Lactobacillus bulgaricus* à hydrolyser, on inocule un lait de vache ou un milieu de culture synthétique avec une culture d'ensemencement de ce microorganisme, et on l'incube durant 3-6 h à 37-45°C.

4. Utilisation selon la revendication 3, dans laquelle on réalise l'hydrolyse à 15-37°C, durant 1-24 h, à l'aide de 1-500 µg de lysozyme par ml d'une culture contenant 10⁶-10¹⁰ cellules de *Lactobacillus bulgaricus* par ml.

5. Utilisation dans la préparation d'un lait fermenté, notamment d'un yogourt, ou dans la préparation d'un produit lactosérique, d'un procédé de préparation d'un agent immunostimulant, dans lequel on hydrolyse avec du lysozyme les peptidoglycanes de la paroi de cellules de *Lactobacillus bulgaricus*, pour en dériver des N-acétyl-muramyl-peptides.

6. Utilisation selon la revendication 5, dans laquelle le lysozyme est un lysozyme de présure, sous forme purifiée ou sous forme de la présure elle-même.

7. Utilisation selon la revendication 5, dans laquelle on inocule le lait ou le produit lactosérique avec une culture d'ensemencement de *Lactobacillus bulgaricus*, et l'on incube durant 3-6 h à 37-45°C.

8. Utilisation selon la revendication 7, dans laquelle on réalise l'hydrolyse à 15-37°C, durant 1-24 h, à l'aide de 1-500 µg de lysozyme par ml de lait ou de produit lactosérique contenant 10⁶-10¹⁰ cellules de *Lactobacillus bulgaricus* par ml.

## Patentansprüche

1. Verwendung eines immunstimulierenden Mittels, umfassend N-Acetylmuramylpeptide, welche durch Hydrolyse mit Lysozym aus Peptidoglycanen der Zellwände von *Lactobacillus bulgaricus* hergestellt werden, in einer fermentierten Milch, insbesondere in einem Joghurt, oder in einem Milchserumprodukt.

2. Verwendung nach Anspruch 1, worin das Lysozym ein Lysozym aus Lab in gereinigter Form oder in Form des Labs selbst ist.

3. Verwendung nach Anspruch 1, in welchem zur Herstellung der zu hydrolysierenden Zellen von *Lactobacillus bulgaricus* eine Kuhmilch oder ein synthetisches Kulturmedium mit einer Impfkultur dieses Mikroorganismus beimpft und diese während 3 bis 6 Stunden bei 37 bis 45°C inkubiert werden.

4. Verwendung nach Anspruch 1, worin die Hydrolyse bei 15°C bis 37°C während 1 bis 24 Stunden mittels 1 µg bis 500 µg Lysozym pro ml einer Kultur ausgeführt wird, welche 10⁶ bis 10¹⁰ Zellen *Lactobacillus bulgaricus* pro ml enthält.

5. Verwendung eines Verfahrens zur Herstellung eines immunstimulierenden Mittels, worin die Peptidoglycane der Zellwände von *Lactobacillus bulgaricus* mit Lysozym hydrolysiert werden, um N-Acetylmuramylpeptide zu erhalten, in der Herstellung einer fermentierten Milch, insbesondere eines Joghurts, oder in der Herstellung eines Milchserumproduktes.

6. Verwendung nach Anspruch 5, worin das Lysozym ein Lysozym aus Lab in gereinigter Form oder in Form des Labs selbst ist.

7. Verwendung nach Anspruch 5, worin die Milch oder das Milchserumprodukt mit einer Impfkultur von *Lactobacillus* beimpft und diese während 3 bis 6 Stunden bei 37 bis 45°C inkubiert werden.

8. Verwendung nach Anspruch 7, in welchem die Hydrolyse bei 15°C bis 37°C während 1 bis 24 Stunden mittels 1 bis 500 µg Lysozym pro ml Milch oder Milchserumprodukt ausgeführt wird, die bzw. das 10⁶ bis 10¹⁰ Zellen *Lactobacillus bulgaricus* pro ml enthält.

## Claims

1. Use in a fermented milk, in particular in a yoghurt, or in a whey-based product, of an immunostimulant agent comprising N-acetyl-muramyl-peptide derivatives, by hydrolysis with the lysozyme, of the peptidoglycanes of the wall of cells of *Lactobacillus bulgaricus.*

2. Use according to claim 1, wherein the lysozyme is a rennin lysozyme, in purified form or in the form of rennin itself.

3. Use according to claim 1 wherein, in order to prepare the cells of *Lactobacillus bulgaricus* to be hydrolysed, cow's milk or a synthetic culture medium is inoculated with a seeding culture of this microorganism, and is incubated for 3-6 h at 37-45°C.

4. Use according to claim 3, wherein hydrolysis is carried out at 15-37°C for 1-24 h with the aid of 1-500 *µ*g of lysozyme per ml of a culture containing 10⁶-10¹⁰ cells of *Lactobacillus bulgaricus* per ml.

5. Use in the preparation of a fermented milk, in particular a yoghurt, or in the preparation of a whey-based product, of a process for preparing an immunostimulant agent, wherein the peptidoglycanes of the wall of cells of *Lactobacillus bulgaricus* are hydrolysed with lysozyme, so as to prepare derivatives of the N-acetyl-muramyl-peptides thereof.

6. Use according to claim 5, wherein the lysozyme is a rennin lysozyme, in purified form or in the form of rennin itself.

7. Use according to claim 5, wherein milk or a whey-base product is inoculated with a seeding culture of *Lactobacillus bulgaricus,* and is incubated for 3-6 h at 37-45°C.

8. Use according to claim 7, wherein hydrolysis is carried out at 15-37°C for 1-24 h, with the aid of 1-500 *µ*g of lysozyme per ml of milk or of a whey-based product containing 10⁶-10¹⁰ cells of *Lactobacillus bulgaricus* per ml.
